# EUROPEAN PATENT APPLICATION

(11) **EP 2 048 502 A1**
(43) Date of publication of application: **15.04.2009**
(21) Application number: 07790764.0
(22) Date of filing: 13.07.2007
(51) Int. Cl.: G01N 33/543, G01N 33/53, C12N 15/09

(54) **METHOD OF CONFIRMING THE SPECIMEN-DETECTING ACTIVITY OF MICROPARTICLES**

(30) Priority: 13.07.2006 JP 2006192741
(71) Applicant: Olympus Corporation, Tokyo 151-0072 (JP)
(72) Inventor: NISHIKAWA, Kazutaka, Tokyo 151-0072 (JP)
(74) Representative: von Hellfeld, Axel
(86) International application number: PCT/JP2007/063983
(87) International publication number: WO 2008/007773

(57) **Abstract**

The present invention provides: a method of testing the performance of reagents containing microparticles by mixing a standard specimen containing two or more kinds of ligands with two or more kinds of microparticles containing receptors capable of specifically binding to the respective ligands and being optically distinguishable, and detecting the optical characteristics of the aggregates thus formed; a kit for detecting a specimen provided with the standard specimen and the microparticles as described above; a method of quantifying a specimen after testing the performance of reagents containing microparticles as described above, mixing a specimen containing two or more kinds of ligands capable of specifically binding to the respective receptors in the microparticles with the microparicles, detecting the optical characteristics of the aggregates thus formed, and then repeating the testing performance of reagents containing microparticles. Thus the present invention provides a performance test of reagents containing microparticles which is suitable for detecting a biological specimen such as a nucleic acid, a quantification method of a specimen which uses the performance testing method thereof and a specimen detection kit including the reagent.

## Description

### TECHNICAL FIELD

The present invention relates to a method for testing performance of reagents containing microparticles which is suitable for detecting a specimen, such as a nucleic acid or the like, a quantification method of a specimen in which the performance testing method is applied thereto, and a kit for detecting a specimen including the microparticles.
The present application is based on Japanese Patent Application No. 2006-192741, filed on July 13, 2006, the entire contents of which are incorporated herein by reference.

### BACKGROUND ART

As a method of detecting a specimen as a target object, a method in which the specimen is specifically bound to a dispersible microparticle has been conventionally known. For example, a protein quantifying method has been known which uses a protein of known concentration as a standard specimen (i.e. Patent Document 1). The example thereof includes a blood test performed as part of a medical checkup or the like, such that a standard serum is used as a standard specimen. Furthermore, a specimen detection method with the specimen being nucleic acids has been suggested (i.e. Patent Document 2). However, a standard specimen is not specified unlike the protein quantifying method described above. Furthermore, some such methods use a synthetic polynucleotide as a standard specimen in a nucleic acid amplification step (i.e. Patent Document 3), and in a gene expression analysis, a preparation method of a standard specimen based on a genomic DNA has been reported with the specimen being nucleic acids. In the specimen detection method thereof, it is important for a specimen detection reagent to exhibit normal detection activity.
[Patent Document 1] Japanese Unexamined Patent Application Publication No. Hei 6-167495
[Patent Document 2] Japanese Patent No. 3545158
[Patent Document 3] Japanese Unexamined Patent Application Publication No. 2003-265190
[Patent Document 4] Japanese Unexamined Patent Application Publication (Translation of PCT Application) No. 2004-532034

### DISCLOSURE OF THE INVENTION

### [Problems that the Invention is to Solve]

However, the standard specimens described in Patent Documents 3 and 4 are used for the quantification of amplified nucleic acids in a gene amplification method such as real-time PCR or the like. Further, various detection signals are employed; for example, fluorescent light, emission of light, and signals obtained from electrophoresis, aggregation, absorbance and the like, depending on the type of a standard specimen. The methods described in Patent Documents 3 and 4 require configuring the detection method and the system so as to have limited versatility. Furthermore, as for a nucleic acid as a specimen which specifically binds to a dispersible microparticle for being detected, a suitable standard specimen candidate has not been reported yet. In addition, when a gene which is retrieved from a biological body is used as a standard specimen, there are possibilities of degradation, a difficulty of purification and contamination depending on storage and the detecting conditions.
As described above, there have been no suitable standard specimen candidates for detecting nucleic acids with a wide versatility available yet. Therefore, there have been problems concerning the difficulty of confirming whether or not a reagent exhibits a normal activity for detecting a standard specimen and a specimen, and the difficulty of correcting the detected value of the nucleic acids. Therefore, the problems described above can be overcome if a standard specimen which is not prone to degradation, contamination or the like, a simple detection method and a detection kit is available.

The present invention was conceived in view of the above-described circumstances, and has as its objective the provision of a method of testing performance of reagents containing microparticles which is suitable for detecting a biological specimen such as nucleic acid or the like, a specimen quantifying method using the performance testing method, and a specimen detecting kit including the reagent used for the above methods.

### [Means for Solving the Problems]

Therefore, in order to accomplish the above object, a first aspect of the present invention is a method for testing the performance of reagents containing microparticles, comprising the following steps: mixing a standard specimen containing a plurality of types of ligands and a plurality of types of microparticles which are optically different containing a receptor which binds to a specific type of ligand; and detecting the optical characteristics of formed aggregates.
A second aspect of the present invention is the method for testing the performance of reagents containing microparticles according to the first aspect of the present invention, wherein the standard specimen and the microparticles are mixed in a buffer.
A third aspect of the present invention is the method for testing the performance of reagents containing microparticles according to the second aspect of the present invention, wherein the standard specimen and the buffer are mixed and the microparticles are added into a mixture of the standard specimen and the buffer.
A forth aspect of the present invention is the method for testing the performance of reagents containing microparticles according to the second aspect of the present invention, wherein the microparticles and the buffer are mixed and the standard specimen are added into a mixture of the microparticles and the buffer.
A fifth aspect of the present invention is the method for testing the performance of reagents containing microparticles according to any one of the first to forth aspects of the present invention, wherein the microparticle is a magnetic microparticle and a magnetic force is applied to a formed aggregate.
A sixth aspect of the present invention is the method for testing the performance of reagents containing microparticles according to any one of the first to fifth aspects of the present invention, wherein the standard specimen and the microparticle are mixed at a temperature in the range of 4-40°C.
A seventh aspect of the present invention is a method for testing the performance of reagents containing microparticles, wherein a detected value of a target standard specimen detected by the performance test of reagents containing microparticle according to any one of the first to sixth aspects of the present invention is compared to a standard specimen of known concentration and exhibiting the same type to the target standard specimen.
A eighth aspect of the present invention is a method for testing the performance of reagents containing microparticles, wherein the method for testing the performance of reagents containing microparticles according to any one of the first to sixth aspects of the present invention is performed over a desirable time course by using a standard specimen of known concentration.

A ninth aspect of the present invention is a specimen quantification method comprising the following steps: testing the performance of reagents containing microparticles by using a method described in any one of the first to sixth aspects of the present invention; mixing a standard specimen containing a plurality of types of ligands and a plurality of types of microparticles which are optically different and contain a receptor which binds to a specific ligand; detecting the optical characteristics of formed aggregates; and repeating the performance test of reagents containing microparticles.
A tenth aspect of the present invention is a specimen quantification method comprising the following steps: testing performance of reagents containing microparticles with a standard specimen of the same type and a plurality of different known concentrations; deriving a function of the detected values and the concentrations of the standard specimen; and quantifying the specimen from the function and a detected value of a specimen obtained when the specimen containing the same type of ligand to that containing in the standard specimen and the microparticle are mixed.

A eleventh aspect of the present invention is a specimen detection kit comprising: a standard specimen containing a plurality of types of ligands and a plurality of types of optically different microparticles containing a receptor which binds to a specific ligand.
A twelfth aspect of the present invention is a specimen detection kit comprising: a standard specimen mixture containing a plurality of types of the standard specimens and each of the standard specimens containing a plurality of types of ligands, and a plurality of types of optically different microparticles containing a receptor which binds to a specific ligand.
A thirteenth aspect of the present invention is the specimen detection kit according to the eleventh or twelfth aspect of the present invention, wherein the ligand containing in the standard specimen is hapten.
A fourteenth aspect of the present invention is the specimen detection kit according to any one of the eleventh to thirteenth aspects of the present invention, wherein a type of the ligand contained in the standard specimen is the same type as that contained in a specimen.
A fifteenth aspect of the present invention is the specimen detection kit according to any one of the eleventh to thirteenth aspects of the present invention, wherein the number and the type of ligand contained in the standard specimen is the same as the ligand contained in a testing specimen.
A sixteenth aspect of the present invention is the specimen detection kit according to any one of the eleventh to fifteenth aspects of the present invention, wherein the type of the standard specimen is the same as the testing specimen.
A seventeenth aspect of the present invention is the specimen detection kit according to any one of the eleventh to sixteenth aspects of the present invention, wherein the molecular weight of the ligand contained in the standard specimen is within the range of 180-60000.
A eighteenth aspect of the present invention is the specimen detection kit according to any one of the eleventh to seventeenth aspects of the present invention, wherein the ligand contained in the standard specimen binds to a specific receptor contained in the microparticle at a temperature within the range of 0-100°C.
A nineteenth aspect of the present invention is the specimen detection kit according to any one of the eleventh to eighteenth aspects of the present invention, wherein the ligand contained in the standard specimen is a fluorescent substance, digoxigein, a polypeptide containing six or more of amino acids, polysaccharide chain consisting of a plurality of monosaccharide chain, biotin, a protein, a polyhistidine, hyaluronic acid (HA), glutathione S-transferase (GST), a peptide expressed with a sequence number 1 (Flag) and more than one type selected from groups of derivatives of the high molecular compounds listed above.
A twentieth aspect of the present invention is the specimen detection kit according to any one of the eleventh to nineteenth aspects of the present invention, wherein the standard specimen contains a high molecular compound which includes a chain structure.
A twenty-first aspect of the present invention is the specimen detection kit according to the twentieth aspect of the present invention, wherein the high molecular compound is soluble in water.
A twenty-second aspect of the present invention is the specimen detection kit according to the twentieth or twenty-first aspect of the present invention, wherein the high molecular compound is provided with the ligands at a main chain or ends of a side chain of the high molecular compound.
A twenty-third aspect of the present invention is the specimen detection kit according to any one of the twentieth to twenty-second aspects of the present invention, wherein the high molecular compound is polysaccharide, polyethylene glycol, a polynucleotide, polypeptide, a polyacrylic acid derivative, a polyacrylamide, polyester, polyether, a polyamide, or more than one type selected from groups of derivatives of the high molecular compounds listed above.
A twenty-forth aspect of the present invention is the specimen detection kit according to any one of the twentieth to twenty-third aspects of the present invention, wherein the length of the main chain of the high molecular compound is within the range of 15-200 nm.
A twenty-fifth aspect of the present invention is the specimen detection kit according to any one of the twentieth to twenty-forth aspects of the present invention, wherein the high molecular compound is a linier structure in a solution used for mixing the standard specimen and the microparticle.
A twenty-sixth aspect of the present invention is the specimen detection kit according to any one of the eleventh to twenty-fifth aspects of the present invention, wherein the molecular length of the standard specimen is within the range of 80-120% of that of a specimen.
A twenty-seventh aspect of the present invention is the specimen detection kit according to any one of the eleventh to twenty-sixth aspects of the present invention, wherein the standard specimen is provided with known concentrations of a plurality of different values.
A twenty-eighth aspect of the present invention is the specimen detection kit according to the twenty-seventh aspect of the present invention, wherein the plurality of different concentrations are within the range of 1pM-1µM.
A twenty-ninth aspect of the present invention is the specimen detection kit according to any one of the eleventh to twenty- eighth aspects of the present invention, wherein the diameter of the microparticle is within the range of 0.1-10µM.
A thirtieth aspect of the present invention is the specimen detection kit according to any one of the eleventh to twenty- eighth aspects of the present inventions, wherein the microparticle is a magnetic microparticle.
A thirty-first aspect of the present invention is the specimen detection kit according to the thirtieth aspect of the present invention, wherein the diameter of the microparticle is within the range of 0.1-20µM.
A thirty-second aspect of the present invention is the specimen detection kit according to any one of the eleventh to thirty-first aspects of the present invention, wherein a reagent which is used for mixing the standard specimen and the microparticle is provided in the kit.

### [Advantages of the Invention]

According to the present invention, performance of reagents containing microparticles which is preferred for detecting a biological specimen such as nucleic acids or the like, can be confirmed simply with a high precision. Further, the specimen can be detected simply with a high precision. For example, the present invention is suitable for detecting a nucleic acid contained in whole blood and in a frozen or fixed tissue specimen, or a specimen in which nucleic acids is processed. Therefore, the present invention facilitates, for example, simplification and acceleration of clinical laboratory testing. Furthermore, it is also possible to widely apply the present invention from the simplification of testing a specimen to fully automated analysis.

### BRIEF DESCRIPTION OF THE DRAWINGS

FIGS. 1A-1D are schematic diagrams showing preparation methods of standard specimens.
FIGS. 2A and 2B show microparticles in which receptors are bound thereto.
FIG.3 is a schematic diagram showing microparticles bound to standard specimens to form an aggregate.
FIG. 4A is a diagram showing a PCR product in which both of the 5'ends are biotinylated.
FIG. 4B is a diagram showing a PCR product in which both of the 5'ends are FITC-labelled.
FIG. 4C is a diagram showing a PCR product in which one of 5'end is FITC-labelled and another 5'end is biotinylated.

### DESCRIPTION OF REFERENCE NUMERALS AND SIGNS

- 30: Standard specimen
- 31: First ligand
- 32: Second ligand
- 33: First microparticle
- 331: First receptor
- 34: Second microparticle
- 341: Second receptor

### SEQUENCE LISTING

A list of sequences which are used in Example of the present invention is attached.

### BEST MODE FOR CARRYING OUT THE INVENTION

Preferred embodiments of the present invention will be described in detail.
In the present invention, "Ligand" refers to a ligand contained in a standard specimen unless a specific explanation is provided.
In the present invention, 'Standard specimen' refers to an aggregate including more than a plurality types of ligands and mainly consisting of one or a plurality of molecules.
In the present invention, "Microparticle" refers to a dispersive microparticle including a receptor which specifically binds to each of the ligands contained in the standard specimen and employing a plurality types of microparticles which are optically different. More specifically, herein "optically distinguishable" refers to exhibiting, for example, a different color resulting from different wavelength of absorptions, reflection, dispersion, emission or fluorescence of light, and the like; or different diameter sizes, different shapes, different materials employed in the particles, and the like. For example, although the color and concentration of particles are the same, their diameters are different and therefore their optical transmittances would be different. Thus, the particles become optically distinguishable. Furthermore, if the shapes of microparticles are different, the degree of scattering light would also differ; hence the microparticles become optically distinguishable. If microparticles are made of different materials, the surface of the microparticles would exhibit a different physical property; and therefore, the microparticles would also be optically distinguishable. A microparticle used in the present invention can also exhibit a plurality of optically distinguishable characteristics in addition to the characteristics described above. The microparticles bind to the standard specimen via a ligand and a receptor, such that a plurality of the standard specimens are bound to a microparticle, and each of the standard specimens further bind to other microparticles. As a result, a plurality of the microparticles are bound to each other forming an aggregate of the microparticles.
Herein "Binding a standard specimen to a microparticle" refers to a binding via the ligand and the receptor.

In addition, herein "Specific binding" refers to a binding with a highly specific force based on an intermolecular dispersion force formed selectively between specific substances; for example, hybridization of DNA or RNA formed between complementary sequences of a stable double-stranded nucleotide sequences, a binding between an antigen and an antibody, a binding between biotin and avidin, or the like.

By detecting the optical characteristics of a formed aggregate, performance of reagents containing microparticles can be tested for whether or not the reagent is normal in order to detect a specimen. More precisely, the optical characteristics can be detected by, for example, measuring transmittance or absorbance within ultraviolet-visible infrared region, fluorescence assay, luminescence assay, observation with an optical microscope, and the like. The performance of reagents containing microparticles is then determined according to the detected value.

If the performance of reagents containing microparticles is normal, the optical characteristics of the formed aggregate reflect optical characteristics of all of the types of microparticles which should bind to the standard specimen. On the other hand, if the optical characteristics of the formed aggregate do not reflect the optical characteristics of any type of microparticle which should bind to the standard specimen, this indicates an abnormality in the performance of reagents containing microparticles in which the optical characteristics are not reflected. For example, when microparticles with different colors are employed as a plurality of types of the optically distinguishable microparticles, and if there are no abnormalities in the performance of reagents containing microparticles, the color of the formed aggregate will be a color in which colors of all of the microparticles contained in the reagent are mixed. However, if any of the microparticles contained in the reagent have abnormalities, the color of the formed aggregate will not be the mixed color of all of the microparticles contained in the reagent.

As for a standard specimen, various types may be employed according to a detection target; however the most preferred types are biological materials. Herein "Biological material" refers to a substance which is extracted or isolated from a living body, and includs not only a directly extracted substance but also a substance which has been chemically treated or chemically modified. Examples thereof include nucleic acids, proteins, and the like, and those which have been chemically treated or chemically modified. As for the nucleic acids, all types of nucleic acids and nucleic acid analogs including cDNA, genomic DNA, synthetic DNA, mRNA, RNAs, hnRNA, and synthetic RNA may be listed such that the nucleic acids may either be naturally originated or artificially synthesized. As for the proteins, hormones, tumor markers, enzymes, antibodies, antigens, abzymes, proteins provided with a plurality of epitopes, and the like may be listed and the proteins may either be of natural origin or artificially synthesized. The most preferable biological material among the biological materials listed above is a nucleic acid. In addition, aqueous polymers other than those substances listed above may preferably be employed. Herein "Standard specimen" refers to a biological material provided with a ligand which specifically binds to a receptor on a microparticle.

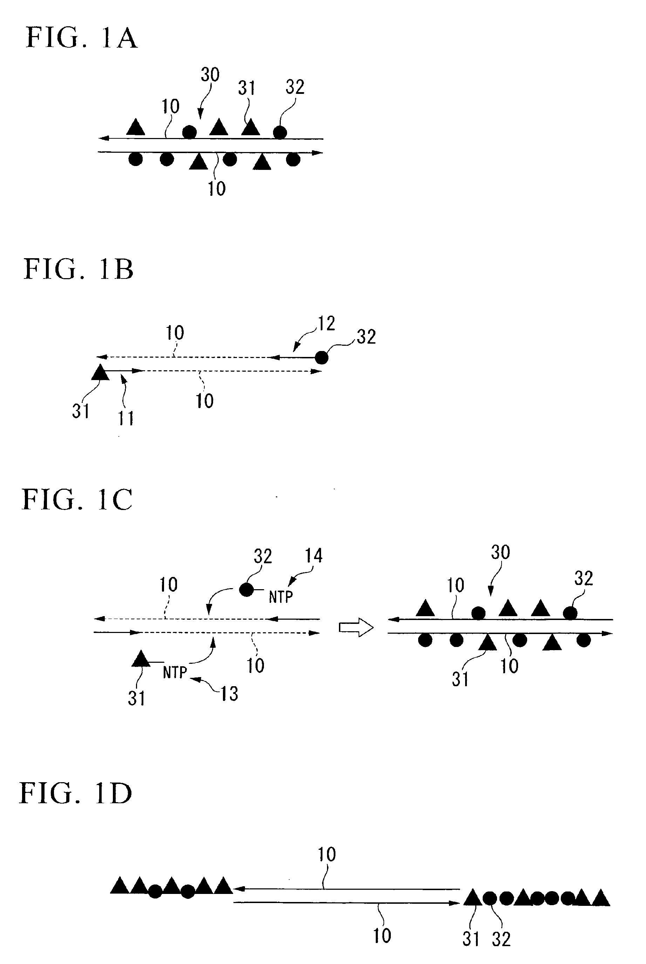

The standard specimen provided with a ligand is prepared with a conventional method.
Examples of preparation methods of a standard specimen 30 (which is a nucleic acid) containing two types of ligands are described below:
(i) a covalent bonding method in which a first ligand 31 and a second ligand 32 are bonded to a base or 5'end of a target nucleic acid 10 by using light or platinum or via a linker (FIG. 1A). An example thereof is a covalent bonding method between a 5'-end of an amino acid base in a nucleic acid and a carboxyl group of a ligand;
(ii) a base sequence elongation method by a PCR reaction using a first primer 11 and a second primer 12 in which the first ligand 31 and the second ligand 32 are respectively bound thereto (FIG. 1 B);
(iii) a base sequence elongation method by a PCR reaction by incorporating at least one of the nucleotide 13 bound to the first ligand 31 and at lease one of the nucleotide 14 bound to the second ligand 32 (FIG. 1C);
(iv) a tailing method of a nucleotide which is bound to the first ligand 31 or the second ligand 32 to a 3'-end of a nucleic acid by using a terminal deoxynucleotidyl transferase (FIG. 1D).

When a sample in which a target nucleic acid exists with other nucleic acids, for example a biological specimen, an optional sample, or the like, the methods of (ii) and (iii) are preferred. Any of the methods may be employed when a target nucleic acid exists by itself. In addition, the target nucleic acid which includes a biological sample, or the like, may be used for the preparation of a standard specimen after amplifying by a PCR reaction, or the nucleic acid may be directly used for the preparation without the amplification step.
FIG. 1 indicates an example of preparing a double stranded nucleic acid including two types of ligands as a standard specimen. However, the methods of (i) and (iv) may also be employed for preparing a single stranded nucleic acid including two types of ligands as a standard specimen. Furthermore, any of the methods of (i)-(iv) can be employed for preparing a nucleic acid including multiple types of ligands.

For example, when an aqueous polymer or the like, other than nucleic acids and protein, are employed as a standard specimen, a standard specimen can be prepared by using a conventional method such as by covalent bonding between a functional group contained in a ligand and a functional group included in the aqueous polymer or the like.

A ligand used in the present invention is not limited as long as it binds specifically to a receptor on a microparticle. Examples of the ligand may include a hydrophilic organic compound, hapten, digoxigein, fluorescein, Alexa, fluorescein isothiocyanate (FITC), 2,4- dinitrophenols (DNP), tetramethyl rhodamine (TAMRA), a polypeptide containing six or more of amino acids, polysaccharide chain consisting of a plurality of monosaccharide chains, biotin, a protein, polyhistidine, hyaluronic acid (HA), glutathione S-transferase (GST), a peptide expressed with a sequence number 1 (Flag) and more than one type selected from groups of derivatives of the high molecular compounds listed above.
Among the groups listed above, biotin, digoxigein, fluorescein, Alexa and DNP have advantages in their availability and their low costs. On the other hand, the sugar chain and the peptide have advantages of having a high degree of freedom in design of the chemical structure; therefore, a variety of types are easily prepared.

The number of ligands in a standard specimen may be one or more per type of ligand, and the number may be adjusted depending on a detection method of a standard specimen. The position of the ligand in a standard specimen is not limited as long as a specific binding between the ligand and the receptor is not prevented; the position can be selected in order to suite for a preparation procedure of a standard specimen. For example, if a standard specimen is a nucleic acid, the suitable position is the 5'-end.
Furthermore, when the standard specimen is an aggregate consisting of a plurality of molecules, a ligand may be included only in one molecule contained in the aggregate, or a ligand may be included in a plurality of molecules in the aggregate.

The temperature range for mixing a standard specimen and a microparticle in order to cause a specific binding to a receptor on a microparticle may preferably be within the range of 1-100°C, more preferably within the range of 4-40°C, and even more preferably within the range of 18-38°C.

As described below, a target specimen can be quantified by using the method for testing performance of reagents containing microparticles in the present invention. In this case, it is more preferable that the type of ligand contained in a standard specimen be the same as that of a ligand contained in a specimen. Furthermore, it is more preferable that a number and a type of ligand in a standard specimen be the same as those of the specimen. Thereby, the quantification of the specimen can be more precisely performed.

It is preferable that a standard specimen contain a high molecular compound which includes a chain structure therein. In particular, although the chain structure may be formed as a part of the standard specimen, it is more preferable that the standard specimen be a high molecular compound in which the entire structure thereof is consisting of a chain structure. Further, a high molecular compound including the ligand at the backbone or the end of the side chain is preferably used due to the ease of preparation and of standardization of the number of ligands contained in the standard specimen.

The examples of the preferred high molecular compound may be listed as: polysaccharide, polyethylene glycol, a polynucleotide, polypeptide, a polyacrylic acid derivative, a polyacrylamide, polyester, polyether, a polyamide, and more than one type selected from groups of derivatives of the high molecular compounds listed above. Examples of the high molecular compounds may include a polyethylene glycol where a ligand is bound to an end of the chain structure, a polypeptide, a polysaccharide, and the like. Furthermore, a synthetic polynucleotide in which a ligand is bound thereto may be preferably employed due to its ease of preparation. As described above, a nucleic acid in which a ligand is bound to the 5'-end is obtained from a PCR reaction using a primer in which the ligand is bound to the 5'-end. Nucleic acids in which a ligand is bound to the 5'-end is one example of a compound in which the preparation and the standardization of the number of ligands contained in the standard specimen is easy.

It is preferable that the length of the main chain of a high molecular compound be within the range of 15-200nm, more preferably within the range of 20-180nm, and the most preferably within the range of 45-100nm. When the backbone is a nucleic acid, it is preferable that the number of nucleobase be less than 500 base pairs (hereinafter abbreviated as "bp"). More specifically, it is preferable that the number of nucleobase be within the range of 50-250bp, more preferably within the range of 55-220bp, and the most preferably within the range of 58-120 bp. The backbone length of the molecule containing a ligand is set as described above in order to facilitate a specific binding between the ligand and a receptor. This is because the backbone length of the molecule containing the ligand influences the specific binding.
Furthermore, as described above, a biological substance is preferable as a standard specimen. When an aqueous substance such as the biological substance is employed as a standard specimen, it is preferable that the high molecular compound described above be soluble in water.
Furthermore, it is preferable that the high molecular compound be a linier structure in a reagent solution used for mixing the standard specimen and microparticles. If the structure is circular (not linier), a ligand may not be exposed easily in the solution, such that the specific binding of the ligand between a receptor may be difficult to form.

It is preferable that a molecular length of a standard specimen be substantially the same as that of a detection target specimen. In particular, it is preferable that a molecular length of a standard specimen be within the range of 80-120% with respect to the molecular length of a detection target specimen, more preferably within the range of 90-110% and the most preferably within the range of 95-105%. By designing the molecular length in this manner, a quantification of the target specimen can be more precisely performed.

When the specific binding is formed between an antigen and an antibody, the size of a ligand needs to be optimized so as to allow the specific binding. For example, it is preferable that the size of the ligand be a molecular weight within the range of 180-60000, more preferably within the range of 180-10000 and the most preferably within the range of 180-1000.

Herein "microparticle" refers to a dispersible microparticle, for example, the microparticle may be a colloid particle which disperses in a solution. A latex particle is preferably used; however, the present invention is not limited thereto. The microparticle also includes a receptor which specifically binds to each ligand contained in a standard specimen.
Examples of a receptor may include, for example an antibody, lectin, streptavidin and the like; however, the present invention is not limited thereto. Proteins, in particular, antibodies are preferably selected as a receptor.

A number of type of a receptor included in a microparticle may be one; however a plurality of types may be designed so as to allow the microparticle to bind to a plurality of types of ligands in a standard specimen.

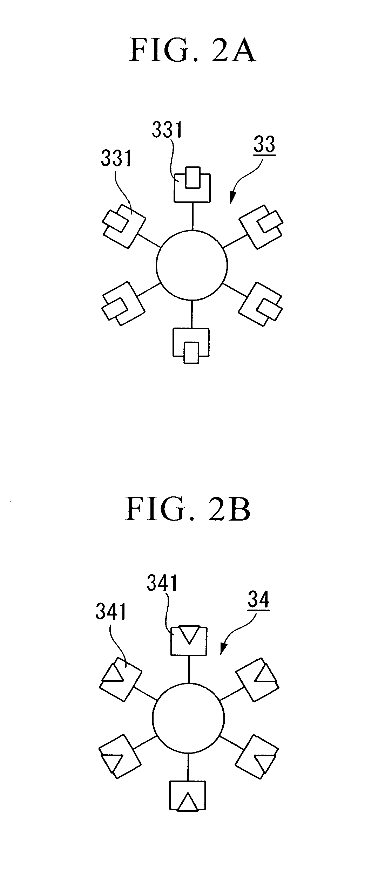

FIG. 2 shows examples of microparticles employed in the present invention. The microparticles illustrated here are microparticles used for a detection of a standard specimen containing a first ligand and a second ligand. FIG. 2A illustrates a first micorparticle 33 which only includes a plurality of a first receptor 331 specifically bound to the first ligand, and FIG. 2B illustrates a second micorparticle 34 which only includes a plurality of second receptors 341 specifically bound to the second ligand. Both FIGS 2A and 2B are plain views illustrating six of the first receptors 331 and the second receptors 341; however, the number of the receptors is not limited to six. Furthermore, the first microparticle 33 and the second microparticle 34 are optically distinguishable from each other.

It is preferable that the diameter of the microparticle be within the range of 0.1-10.0 µm, more preferably within the range of 0.1-1 µm and the most preferably within the range of 0.1-0.35µm. A stable aggregate can be easily obtained with the microparticle having such diameter sizes.

The latex particle mainly consists of polystyrene and methacrylic acid is copolymerized in order to improve hydrophilicity and dispersibility. There are two types of the latex particles: a plain type which does not include a functional group on its surface, and a functional type which includes a functional group on its surface. The surface of the plain type of the latex particle is negatively charged due to the presence of methacrylic acid so as to covalently bond to a positively charged section of a protein molecule. It is also possible to form a hydrophobic bonding between the plain type of the latex particle and a protein molecule. Since the plain type of the latex particle exhibits the characteristic of absorbing a protein by being mixed with a protein, a protein fixing can be easily performed. As for a receptor, various types as described above can be employed; however, the plain type of the latex particle may be preferably selected as a microparticle when various types of protein are used.

The latex particle including a functional group is designed such that a carboxyl, amino groups and the like are exposed outside of the latex particle. Thus, various types of the functional group-typed latex particles can be used. When the latex particle containing a functional group is bound to a receptor, the functional group of the latex particle can be bound to a functional group of the receptor, and a conventional binding method may be employed. Examples of the methods include; a EDAC method which binds carboxylic acid and an amino group directly using water-soluble carbodiimide; a binding method using EDC (1-ethyl-3-[3-dimethylaminopropyl]carbodiimide hydrochloride) and HNS (N-hydroxy succinimide) which are premixed in a reaction solution as crosslinking agents to assist bonding between carboxylic acid and amino group; a cross-linking method to cross link between amino groups using a dipolar linker; a bonding method to bind an activated aldehyde or tosylate with a functional group contained in a receptor; and the like.
Any of above methods may be applied to the present invention so long as the feature of the present invention is not affected; in particular, the EDAC method is more preferably used. Further, in a same manner to the plain type of the latex particle, the latex particle containing a functional group is preferably used when various typed proteins are used as a receptor. In addition, a microparticle other than the latex particle can bind to a receptor by a conventional method depending on a type of the microparticle.

As for microparticles, more than a plurality of types of optically distinguishable microparticles are employed in the present invention, and an optical characteristic to the microparticles can be provided with a conventional method. For example, when microparticles with different colors are employed, each of the microparticles may be formed from pre-colored material, or the microparticles may be coated with different colors after the microparticles are formed from the same colored material. When microparticles with different diameters or shapes are employed, a desirable diameter or the shape of each type of the microparticles may be designed, and the microparticles manufactured accordingly. When microparticles formed of different materials are required, desirable materials may be selected for manufacturing. Furthermore, a surface of the manufactured microparticles with different diameter sizes, shapes and materials may be coated with, for example, a polymer and the like.

A magnetic microparticle may be employed as the microparticle of the present invention. In this case, when a magnetic force is applied to a resultant aggregate, collection of the aggregate, separation of the aggregate from a solution, and removal of contaminants from the aggregate can be performed easily so as to further improve observation (confirmation) of the optical characteristics of the aggregate.
Examples of a magnetic microparticle include: metals such as triiron tetroxide (Fe₃O₄), iron sesquioxide (γ - Fe₂O₃), various ferrites, iron, manganese, nickel, cobalt, chrome and the like; or magnetic microparticles consisting of alloy including cobalt, nickel, manganese and the like; or a hydrophobic polymer such as polystyrene, polyacrylonitrile, polymethacrylonitrile, polymethyl methacrylate, polycapramide, poly ethylene terephthalate; or a hydrophilic polymer such as polyacrylamide, poly methacrylamide, a polyvinylpyrrolidone, a polyvinyl alcohol, poly(2-oxyethyl acrylate), poly(2-oxy ethyl methacrylate), poly(2,3-dioxy propylacrylate), poly(2,3-dioxy propyl methacrylate), polyethyleneglycol methacrylate; or a co-polymer with 2-4 types of the aforementioned monomers such as a latex, gelatine, liposome, or the like; or a particle in which the aforementioned magnetic microparticle is fixed onto a surface of the latex, gelatine, liposome, or the like. The aforementioned methods may be applied for providing an optical characteristic to the magnetic microparticle.

It is preferable that the diameter size be within the range of 0.1-20 µm, more preferably within the range of 0.1-1 µm and the most preferably within the range of 0.1-0.35 µm; however, the diameter size of a magnetic microparticle is not limited thereto.
A bonding method between a magnetic microparticle and a receptor may include physical and chemical methods such as physical adsorption of the receptor to the microparticle, a chemical modification, and the like.

Examples of the physical adsorption methods are, for example, a method in which a receptor is directly adsorbed and fixed to a magnetic microparticle, a method in which a receptor directly bound to other proteins such as albumin and the like, then the receptor is adsorbed and fixed to a magnetic microparticle.
Examples of chemical modification methods are, for example, a method which activates a functional group such as an amino group, a carboxyl group, a mercapto group, a hydroxyl group, an aldehyde group, an epoxy group and the like, which are present on a surface of a magnetic microparticle, then the activated functional group is bound to various functional groups which are present on a receptor to fix the receptor directly on the magnetic microparticle; a fixing method by chemically bonding a magnetic microparticle and a receptor via a spacer molecule; and a chemical bonding method between a protein and a magnetic microparticle after bonding the protein such as albumin and the like to the receptor. Any conventional methods may be applied in the chemical bonding methods described above. Examples of the spacer molecule include alkyl chain, polyethylene glycol, polyoxyethylene, polysaccharide, polyacrylic acid derivative, polyamide, and the like.

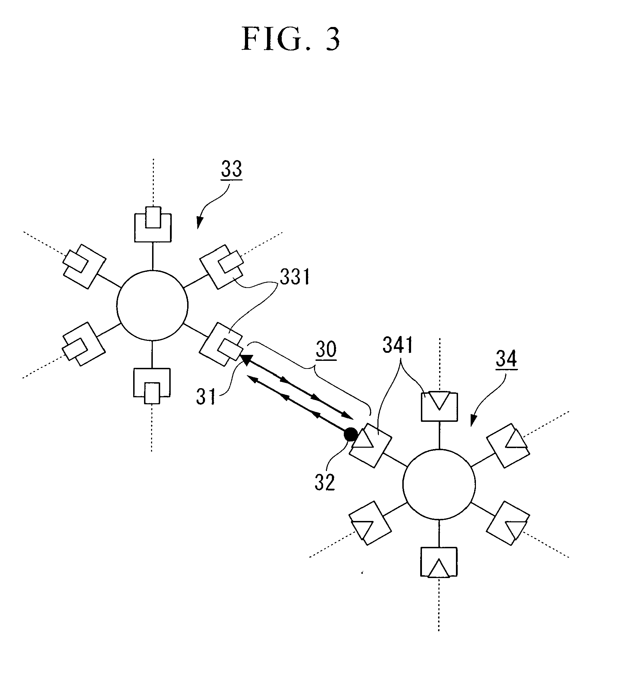

Steps included in a formation of an aggregate when a standard specimen and a microparticle are mixed are explained in detail with FIG.3. In particular, nucleic acids including two types of ligands are used as a standard specimen is taken as an example.
In FIG. 3, the section indicated by the reference number 30 is a standard specimen 30 in which the first ligand 31 is bound to one of the 5'-ends of the standard specimen 30, and the second ligand 32 is bound to another 5'-end of the standard specimen 30. Further, the reference number 33 denotes a first microparticle which only includes a plurality of the first receptors 331 which specifically binds to the first ligand 31. The reference number 34 denotes a second microparticle which only includes a plurality of the second receptors 341 which specifically bind to the second ligand 32.
The standard specimen 30 binds to the first microparticle 33 and the second microparticle 34 via bindings between the first ligand 31 and the first receptors 331; and between the second ligand 32 and the second receptors 341, respectively. Each of the first microparticle 33 and the second microparticle 34 bind to a plurality of standard specimens via the binding between the ligand and the receptor, and the plurality of standard specimens bind to the first microparticle 33 and the second microparticle 34, although the illustration thereof is omitted. Thereby an agglutination of microparticles is formed as a result of a plurality of microparticles binding each other. Note that dotted lines extending from the receptors indicate simplified bound standard specimens. In addition, the number of the first receptors 331 and the second receptors 341 illustrated here is six each; however, the number of the receptors is not limited to six.
Furthermore, the first microparticle 33 and the second microparticle 34 are optically distinguishable microparticles and the formed aggregates reflect the optical characteristics of the microparticles.

If multiple types of standard specimens are available and multiple ligands included in the standard specimens are different depending on each type of the standard specimens, only a microparticle including a receptor which specifically binds to a ligand containing only one of multiple types of the standard specimens is used for the mixing; only that type of the standard specimen can be selectively detected. Similarly, it is possible to prepare a microparticle containing a receptor which specifically binds to multiple types of ligands contained in a standard specimen according to each type of the standard specimens, and the microparticle is used for the mixing. As a result, multiple types of the standard specimens can be detected simultaneously.
As described above, by selecting the microparticle which is used for the mixing depending on a target standard specimen, it is possible to detect a standard specimen for a single specific type or multiple types simultaneously.
Herein, "Detection of a standard specimen or a specimen" refers to detection of the optical characteristics of an aggregate formed by binding the standard specimen or the specimen to a microparticle.

As for the plurality of types of ligands containing the standard specimen, different ligands may be used according to different types of standard specimens as described above. However, the present invention may not be limited thereto; for example, one of the type of ligand among the plurality of types may be used as an universal lignad between a plurality of types of the standard specimens, thereby the detection procedure can be simplified.
Furthermore, an agglutination reaction of microparticles is specific when a plurality of types of the standard specimens are mixed. Therefore, when preferred microparticles are aggregated in a mixture of multiple types of the standard specimens, the agglutination reaction of one of the type of the standard specimen acting as an agglutinin is not prevented by other types of the standard specimen. Therefore, by mixing a plurality of types of the standard specimens, an universal standard specimen which can detect and measure a plurality of types of microparticles can be provided. The universal standard specimen can reduce the number of specimens; hence the burden on a researcher for the preparation can be reduced.

Reaction conditions such as temperature, time and types of reagents used for mixing a standard specimen and a microparticle, may be optimized accordingly, depending on the combination types of lignad-receptor, and the like.
A water-soluble substance such as a biologically related substance is employed as a standard specimen, it is preferable to use an aqueous solution such as a buffer or the like. Examples of the buffer may include phosphoric acid, Tris, HEPES, MOPS, CHARS, sodium acetate, citric sodium, Earle, Hanks, and the like. Furthermore, when the standard specimen is a nucleic acid, the mixing with microparticles is preferably performed at the buffer temperature within the range of 4-40°C, more preferably within the range of 5-30°C, and the most preferably within the range of 18-28°C or 36-38°C. The temperature ranges within 18-28°C is room temperature and the range within 36-38°C is suitable temperatures for a general biological reaction.

It is preferable to mix a standard specimen and a microparticle under the same conditions in order to obtain a highly precise detection value when detection of the standard specimen is performed a plurality of times with the same types of the standard specimen and the microparticle.
On the other hand, by tracking a change of the detected value of a standard specimen over a certain time course by changing the mixing time, a completion time of the agglutination caused by binding between the standard specimens and the microparticles can be determined.

The mixing method of the present invention is not limited so long as it is performed in a solution. For example, mixing a standard specimen and a microparticle may be performed by adding the standard specimen and the microparticle into a solution, or by adding a solution of the standard specimen and a dispersion solution of the microparticle into a solution. The mixing may also simply be performed by mixing the solution of the standard specimen and the dispersion solution of the microparticle. Among the mixing methods above, it is preferable to prepare the standard specimen and the dispersion solution of the microparticle separately, then the two solutions are mixed. When adding the solution of the standard specimen and the dispersion solution of the microparticle into the solution, the solution used in the preparation of the standard specimen and the dispersion solution of the microparticle is preferable to be the same type as the solution in which these solutions are added into. The buffers described above may be preferably employed.

When mixing a standard specimen and a microparticle, if concentrations thereof are too high in the mixing solution, a binding between the standard specimen and the microparticle is prevented, which is known as the "prozone effect". Therefore, in order to detect the optical characteristics of an aggregate with high precision, it is preferable to optimize the concentrations of the standard specimen and the microparticle prior to mixing so as to facilitate a binding between the standard specimen and the microparticle. Preferred methods include for example; a method in which a microparticle is added into a standard specimen solution where the concentration thereof is already optimized with a buffer; or a method in which a standard specimen is added into a dispersion solution of microparticles where the concentration thereof is already optimized with a buffer. It is more preferable that microparticles and a standard specimen be handled as a dispersion solution of the microparticles and a standard specimen solution, respectively.
As for a quantity relationship between a standard specimen and a microparticle, for example, it is preferable that a concentration of the standard specimen be within the rage of 1pM-1µM when a concentration of the microparticles is within the rage of 0.001-0.2 % by mass.

As described above, a performance of reagents containing microparticles can be tested by detecting the optical characteristics of a formed aggregate by mixing the target standard specimen and the microparticle. Further, by comparing the detected value of the target standard specimen to known a detected value of a standard specimen of the same concentration and exhibiting the same type as that of the target standard specimen, a performance of reagents containing microparticles can be confirmed easily.
For example, by comparing the detected values of the target standard specimen obtained for each of a manufactured lot number of microparticles to a detected value of a standard specimen of the same concentration and exhibiting the same type as that of the target standard specimen, the presence or absence of variation in the specimen detection activity of a microparticle between different lot numbers can be confirmed.
Similarly, comparing a detected value of a standard specimen obtained with a microparticle stored at a retailer to a known detected value of a standard specimen which is kept by a manufacture of the microparticle, the presence or absence of abnormality in performance of reagents containing microparticles which is stored by the retailer can be tested. Therefore, use of a defective microparticle can be prevented prior to use and a method of testing performance of reagents containing microparticles of the prevent invention facilitates the quality management of the microparticle.

In addition, abnormality of a performance of reagents containing microparticles can be checked regularly by testing a performance of reagents containing microparticle over a desirable time course with a standard specimen of known concentration. The time for conducting the test may be adjusted according to a purpose.
In this case, for example, when optically different microparticles employed in the detection are used individually and a detected value by the individual microparticles is confirmed in conjunction to the performance test of a reagent, and if a reduction of the performance is observed, cause of the reduction can be specified as to which of the microparticles are caused from.

A specimen detection kit of the present invention includes a standard specimen containing a plurality of types of ligands and a plurality of types of optically different microparticles containing a receptor which specifically binds to each of the ligands. The microparticles and the standard specimen contained in the kit are preferable to be in a form of a dispersion solution of the microparticle and a standard specimen solution, respectively.
Further, it is preferable that a solution used for mixing the microparticles and the specimen solution be provided.
By using the specimen detection kit described above, a preparation step of microparticles and a standard specimen at every detection is eliminated, hence steps in the method of testing performance of reagents containing microparticles and the quantification method of a specimen can be simplified.

It is preferable that the specimen detection kit include microparticles and a standard specimen with a plurality of known concentrations. In this case, it is preferable that the concentration of the standard specimen be prepared within the range of 1pM-1µM which is a highly used concentration range; more preferable concentration of the standard specimen may be within the range of 10pM-500nM, and the most preferable concentration is within the range of 100pM-100nM.
The specimen detection kit facilitates not only a significant reduction of steps in the method of testing performance of reagents containing microparticles and the quantification method of a specimen, but also reduction of product variations of the reagents due to, for example, a variation of concentrations upon preparation of microparticles and a standard specimen contained in the reagents. Furthermore, since different concentrations of microparticles and standard specimens are provided with individual packaging, risk of contamination and degradation can also be reduced.

Furthermore, by employing the method of testing performance of reagents containing microparticles in the present invention, a specimen can be precisely quantified. After confirming the performance of reagents containing microparticles, a standard specimen containing a plurality of types of ligands which specifically binds to a receptor formed on microparticles and the microparticles are mixed, optical characteristics of a resultant aggregate are detected, and the performance testing of reagents is repeated.
If the detected value of the standard specimen is normal before and after the detection of the specimen, the quantification of a specimen can be assured such that it is performed with a high precision with the method of testing performance of reagents containing microparticles of the present invention.

Furthermore, performance of reagents containing microparticles may be confirmed with a standard specimen of the same type and a plurality of different known concentrations, and a function of the detected values and the concentrations of the standard specimen may be derived. Thereby, a specimen can be quantified from the function and a detected value of a specimen obtained when the specimen containing the same type of ligand to that contained in the standard specimen which is used for deriving the function and the microparticles are mixed. At this time, it is preferable that a number of each type of the ligands contained in a specimen be the same as that contained in the standard specimen. Furthermore, it is preferable that a molecular length of the standard specimen be substantially the same as that of the specimen.

The type of the specimen used for the quantification may be different from that of the standard specimen, however the same type as the standard specimen is preferred. This is because the agglutination step becomes the same, hence the detection becomes more precise. The detection of the optical characteristics of the aggregate when the specimen is used can be performed in a similar manner to when the standard specimen is used. Therefore, redundant explanations thereof are omitted.

### [Examples]

To illustrate the present invention in greater detail, the following Examples will be given. However, these Examples are not to be viewed as limiting.

### Example 1:

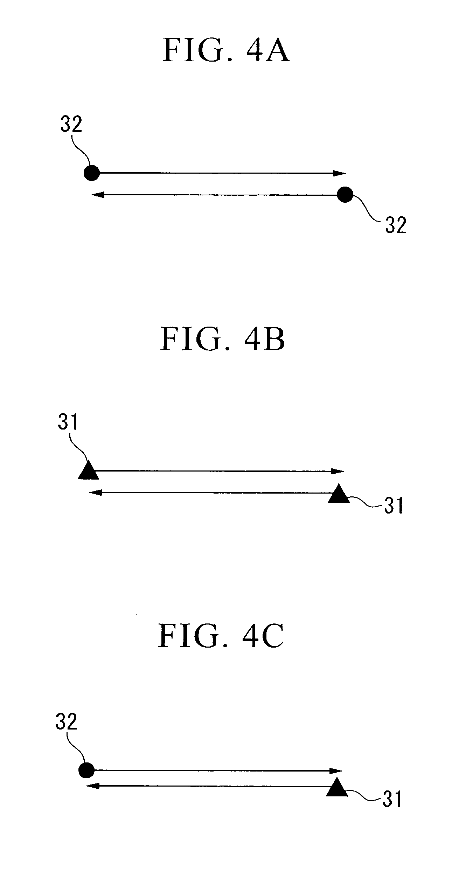

(1) A biotinylated primer with the sequence indicated in the sequence number 2 having the 5'-end biotinylated, a FITC labeled primer with the sequence indicated in the sequence number 3 having the 5'-end bound to FITC, and a synthesized DNA fragment of 56 bp length as a template with the sequence indicated in the sequence number 4 were mixed to be used for a PCR reaction. PCR products in which both 5'-ends were biotinylated, PCR products in which both 5'-ends were labeled with FITC, and one of the 5'-ends was biotinylated and the other end was labeled with FITC were obtained. The concentration of each PCR product was adjusted with Tris buffer to the following concentrations: 100 pM, 1 nM, 10 nM, 100 nM and 1 µM. The PCR products in which both 5'-ends were biotinylated and the PCR products in which both 5'-ends were labeled with FITC were mixed, and the concentrations of each of the products was adjusted to the aforementioned concentrations. The PCR products were used as a specimen to be detected.
   As shown in FIGS. 4A, 4B and 4C, two types of ligands were labeled to both ends of a substance which is a specimen. FIG. 4A shows a PCR product in which both 5'-ends are biotinylated, FIG. 4B shows a PCR product in which both 5'-ends are FITC-labeled, and FIG. 4C shows a PCR product in which one of the 5'-ends is biotinylated and the other 5'-end is FITC-labeled. The substance in which both of the 5'-ends are labeled with the same ligands as shown in FIGS. 4A and 4B can be used as a standard specimen in the same manner as shown in the substance shown in FIG. 4C.
(2) Anti-biotin antibody modified microparticle (P1) with a main absorption wavelength of 800 nm and anti-FITC antibody modified microparticle (P2) with a main absorption wavelength of 450 nm were diluted with a reaction buffer solution (a PBS buffer with pH 7.2) to 0.04 % by mass and 100 µl of the diluted solution was pipetted onto a 96-well plate.
(3) The absorbance of the solution was measured at 450 nm and 800 nm.
(4) The PCR product (5 µl ) in which both ends were biotinylated (shown in FIG. 4A) were added into each well containing the beads solution P1, and 5 µl of the PCR product in which both ends were FITC-labeled (shown in FIG. 4B) were added into each well containing the beads solution P2. The solutions contained in each well were mixed by pipetting. Likewise, 5 µl of a mixture of the PCR products in which both ends were biotinylated (shown in FIG. 4A) and the PCR products in which both ends were FITC-labeled (shown in FIG. 4B) were also added into each well containing the beads solution P 1 or P2 and mixed by pipetting.
(5) The absorbance of the solution which stood for 5 minutes after the addition of the PCR product was measured at 450 nm and 800 nm.
(6) Absorbance differences between (3) and (5) were calculated and results are shown in Table 1.
(7) A mixture of P1 and P2 was diluted with a reaction buffer solution (a PBS buffer with the pH 7.2) to 0.04 % by mass, and 100 µl of the diluted solution were pipetted into a 96-well plate.
(8) An absorbance of the solution was measured at 650 nm.
(9) The PCR product (5 µl) in which one of the 5'-ends was biotinylated and the other end was FITC-labeled (shown in FIG. 4C) was added into each well containing the beads solution P1 and P2. The solutions contained in each well were mixed by pipetting.
(10) The absorbance of the solution which stood for 5 minutes after the addition of the standard specimen was measured at 650 nm.
(11) Absorbance differences between (8) and (10) were calculated and results are shown in Table 1.
(12) The bead mixture containing 100nM of the PCR product among all of the absorbance measured beads mixtures was further incubated for one month at 37°C, and an absorbance difference of the incubated mixture was measured in the same manner described in (6) and (11). The result is shown in Table 2.
(13) Furthermore, for the PCR product obtained in (1) above in which one of the 5'-ends was biotinylated and the other 5'-end was FITC-labeled (FIG. 4C), the concentration was adjusted to 10 nM, 5 nM, 2.5 nM and 1.25 nM.
(14) A PCR product of unknown concentration was obtained by using the method and the material as described in (1).
(15) The absorbance of the PCR products obtained in the steps (13) and (14) was measured with the same method as described in the steps (7)-(11) above. Furthermore, the measurement of absorbance of the 10nM PCR product was repeated. The result is as shown below.

**TABLE 1:**

| PCR product concentrations | Absorbance difference | | | | |
|---|---|---|---|---|---|
| | 800 nM | | 450 nM | | 650 nM |
| | P1 | P2 | P1 | P2 | P1 + P2 |
| 100 pM | 0.0552 | 0.0006 | 0.0398 | 0.0024 | 0.0513 |
| 1 nM | 0.1044 | 0.0017 | 0.0643 | 0.0454 | 0.1333 |
| 10 nM | 0.4329 | 0.0005 | 0.0556 | 0.1882 | 0.5527 |
| 100 nM | 0.7654 | 0.0004 | 0.0528 | 0.3328 | 0.9774 |
| 1 µM | 0.9600 | 0.0021 | 0.0486 | 0.4174 | 1.2259 |

**TABLE 2:**

| Absorbance measuring time | Absorbance difference | | | | |
|---|---|---|---|---|---|
| | 800 nM | | 450 nM | | 650 nM |
| | P1 | P2 | P1 | P2 | P1 + P2 |
| Immediately after the PCR product preparation | 0.7654 | 0.0004 | 0.0528 | 0.3328 | 0.9774 |
| After one month of incubation | 0.5350 | 0.0011 | 0.0498 | 0.3276 | 0.6832 |

**TABLE 3:**

| PCR product concentrations | Absorbance difference |
|---|---|
| | 650 nm |
| | P1 + P2 |
| 10 nM | 0.5544 |
| 5 nM | 0.3014 |
| 2.5 nM | 0.1968 |
| 1.25 nM | 0.1152 |
| PCR product specimen with unknown concentration | 0.4522 |
| 10 nM (repeated measurement) | 0.5490 |

From the result obtained in Table 1, the performance of reagents containing the Anti-biotin antibody modified microparticles (P1) and the anti-FITC antibody modified microparticles (P2) were confirmed to be normal from the performance testing method of the present invention by using the PCR product as a standard specimen in which one of the 5'-ends was biotinylated and the other end was FITC-labeled, and the microparticles P1 and P2.
Furthermore, from the results shown in Table 2, since the absorbance change of P1+P2 decreased after one month of incubation, it was speculated that the performance of the reagents of the microparticles decreased. Furthermore, comparing the absorbance change after one month of the incubation to that of the immediately after preparation of the PCR product, a significant decrease in the absorbance was observed from the microparticles P1 after one month of the incubation, whereas the absorbance of the microparticles P2 did not substantially change after one month of the incubation. Thus, the decrease of the performance of the beads (microparticles) reagent was confirmed to be influenced by the microparticles P1. As described above, the cause of the decrease in the performance of reagents was determined for which types of the microparticles was responsible, by comparing the measurements (detected values) of the optically distinguishable microparticles employed for the performance test of the reagent. The optically distinguishable microparticles were used individually for the performance test.
Furthermore, the relationship between the PCR concentration and the change of the absorbance was: y = 20.3x - 1.23 (where y = PCR product concentration [nM], x = change in absorbance), derived from the results shown in Table 3. Therefore, by substituting the absorbance of the PCR product with unknown concentration, the concentration can be calculated to be 7.95nM. Thus, a specimen of unknown concentration was quantified from the function of the derived PCR product concentration and change in absorbance.
In addition, when the measurement of the absorbance of the 10nM specimen was repeated after measuring the specimen with unknown concentration, the second measurement of the 10nM specimen showed substantially the same value as that of the first measurement. Accordingly, it was possible to indirectly confirm the performance of the microparticles (P1+P2) to be normal before and after the detection of specimen with unknown concentration.
In addition, the measurement results when two types of the standard specimens were mixed are summarized in Table 4 below. The change in absorbance of the microparticles by a specific reaction when two types of the standard specimens including a first specimen in which both of the 5'-ends were biotinylated and a second specimen in which both 5'-ends were FITC-labeled were mixed resulted in the same trend when one type of the standard specimen was used by itself.
Thereby, the results confirmed that the same effect is obtained when a mixture of a plurality of types of standard specimens containing different ligands was used to that when one type of a standard specimen containing only one type of ligand was used by itself.

**TABLE 4**

| PCR product concentrations | Absorbance difference | |
|---|---|---|
| | 800 nm | 450 nm |
| | P1 | P2 |
| 100 pM | 0.0535 | 0.0022 |
| 1 nM | 0.0974 | 0.0427 |
| 10 nM | 0.4126 | 0.1750 |
| 100 nM | 0.7144 | 0.3176 |
| 1 µM | 0.8832 | 0.3875 |

### INDISTRIAL APPLICABILITY

The present invention facilitates a simplification, and acceleration of clinical laboratory testing; thereby it can be widely applied in medical fields.

## Claims

1. A method for testing the performance of reagents containing microparticles, comprising the following steps:
mixing a standard specimen containing a plurality of types of ligands and a plurality of types of microparticles which are optically different containing a receptor which binds to a specific type of ligand; and
detecting the optical characteristics of formed aggregates.

2. The method for testing the performance of reagents containing microparticles according to claim 1, wherein the standard specimen and the microparticles are mixed in a buffer.

3. The method for testing the performance of reagents containing microparticles according to claim 2, wherein the standard specimen and the buffer are mixed and the microparticles are added into a mixture of the standard specimen and the buffer.

4. The method for testing the performance of reagents containing microparticles according to claim 2, wherein the microparticles and the buffer are mixed and the standard specimen are added into a mixture of the microparticles and the buffer.

5. The method for testing the performance of reagents containing microparticles according to any one of claims 1-4, wherein the microparticle is a magnetic microparticle and a magnetic force is applied to a formed aggregate.

6. The method for testing the performance of reagents containing microparticles according to any one of claims 1-5, wherein the standard specimen and the microparticle are mixed at a temperature in the range of 4-40°C.

7. A method for testing the performance of reagents containing microparticles, wherein a detected value of a target standard specimen detected by the performance test of reagents containing microparticle according to any one of claims 1-6 is compared to a standard specimen of known concentration and exhibiting the same type to the target standard specimen.

8. A method for testing the performance of reagents containing microparticles, wherein the method for testing the performance of reagents containing microparticles according to any one of claims 1-6 is performed over a desirable time course by using a standard specimen of known concentration.

9. A specimen quantification method comprising the following steps:
testing the performance of reagents containing microparticles by using a method described in any one of claims 1-6;
mixing a standard specimen containing a plurality of types of ligands and a plurality of types of microparticles which are optically different and contain a receptor which binds to a specific ligand;
detecting the optical characteristics of formed aggregates; and
repeating the performance test of reagents containing microparticles.

10. A specimen quantification method comprising the following steps:
testing the performance of reagents containing microparticles with a standard specimen of the same type and a plurality of different known concentrations;
deriving a function of the detected values and the concentrations of the standard specimen; and
quantifying the specimen from the function and a detected value of a specimen obtained when the specimen containing the same type of ligand to that containing in the standard specimen and the microparticle are mixed.

11. A specimen detection kit comprising:
a standard specimen containing a plurality of types of ligands and a plurality of types of optically different microparticles containing a receptor which binds to a specific ligand.

12. A specimen detection kit comprising:
a standard specimen mixture containing a plurality of types of the standard specimens and each of the standard specimens containing a plurality of types of ligands, and a plurality of types of optically different microparticles containing a receptor which binds to a specific ligand.

13. The specimen detection kit according to claims 11 or 12, wherein the ligand containing in the standard specimen is hapten.

14. The specimen detection kit according to any one of claims 11-13, wherein a type of the ligand contained in the standard specimen is the same type as that contained in a specimen.

15. The specimen detection kit according to any one of claims 11-13, wherein the number and the type of ligand contained in the standard specimen is the same as the ligand contained in a testing specimen.

16. The specimen detection kit according to any one of claims 11-15, wherein the type of the standard specimen is the same as the testing specimen.

17. The specimen detection kit according to any one of claims 11-16, wherein the molecular weight of the ligand contained in the standard specimen is within the range of 180-60000.

18. The specimen detection kit according to any one of claims 11-17, wherein the ligand contained in the standard specimen binds to a specific receptor contained in the microparticle at a temperature within the range of 0-100°C.

19. The specimen detection kit according to any one of claims 11-18,
wherein the ligand contained in the standard specimen is a fluorescent substance, digoxigein, a polypeptide containing six or more of amino acids, polysaccharide chain consisting of a plurality of monosaccharide chain, biotin, a protein, a polyhistidine, hyaluronic acid (HA), glutathione S-transferase (GST), a peptide expressed with a sequence number 1 (Flag) and more than one type selected from groups of derivatives of the high molecular compounds listed above.

20. The specimen detection kit according to any one of claims 11-19, wherein the standard specimen contains a high molecular compound which includes a chain structure.

21. The specimen detection kit according to claim 20, wherein the high molecular compound is soluble in water.

22. The specimen detection kit according to claim 20 or 21, wherein the high molecular compound is provided with the ligands at a main chain or ends of a side chain of the high molecular compound.

23. The specimen detection kit according to any one of claims 20-22, wherein the high molecular compound is polysaccharide, polyethylene glycol, a polynucleotide, polypeptide, a polyacrylic acid derivative, a polyacrylamide, polyester, polyether, a polyamide, or more than one type selected from groups of derivatives of the high molecular compounds listed above.

24. The specimen detection kit according to any one of claims 20-23, wherein the length of the main chain of the high molecular compound is within the range of 15-200 nm.

25. The specimen detection kit according to any one of claims 20-24, wherein the high molecular compound is a linier structure in a solution used for mixing the standard specimen and the microparticle.

26. The specimen detection kit according to any one of claims 11-25, wherein the molecular length of the standard specimen is within the range of 80-120% of that of a specimen.

27. The specimen detection kit according to any one of claims 11-26, wherein the standard specimen is provided with known concentrations of a plurality of different values.

28. The specimen detection kit according to claim 27, wherein the plurality of different concentrations are within the range of 1pM-1µM.

29. The specimen detection kit according to any one of claims 11-28, wherein the diameter of the microparticle is within the range of 0.1-10µM.

30. The specimen detection kit according to any one of claims 11-29, wherein the microparticle is a magnetic microparticle.

31. The specimen detection kit according to claim 30, wherein the diameter of the microparticle is within the range of 0.1-20µM.

32. The specimen detection kit according to any one of claims 11-31, wherein a reagent which is used for mixing the standard specimen and the microparticle is provided in the kit.


SEQUENCE LISTING
<110>Olympus Corporation
<120> METHOD FOR TESTING PERFORMANCE OF REAGENTS CONTAINING MICROPARTICLES
<130>07P01779
<160>4
<210>1
<211>8
<212>PRT
<213>human
<400>1
Asp Tyr Lys Asp Asp Asp Asp Lys
1               5

<210>2
<211>17
<212>DNA
<213>Artificial Sequence
<220>
<223>Designed DNA fragment to act as a primer in PCR method for amplification of
standard sequence:SEQ ID No.4.
<400>2
gtaagggcgg gcctatt 17

<210>3
<211>23
<212>DNA
<213>Artificial Sequence
<220>
<223>Designed DNA fragment to act as a primer in PCR method for amplification of
standard sequence:SEQ ID No.4.
<400>3
gctcagcact gtggatggac cta 23

<210>4
<211>56
<212>DNA
<213>Artificial Sequence
<220>
<223>Designed DNA fragment as standard sequence amplified by primers SEQ ID NO:2
and NO:3 in PCR method.
<400>4
gctcagcact gtggatggac ctaatttcga ttcttcagaa ataggcccgc ccttac 56